(19) Europäisches Patentamt
European Patent Office
Office européen des brevets

(11) Publication number: 0 335 717
A2

(12) # EUROPEAN PATENT APPLICATION

(21) Application number: 89303155.9

(22) Date of filing: 30.03.89

(51) Int. Cl.[4]: C 07 D 493/10
A 61 K 31/35
//(C07D493/10,323:00,311:00)

(30) Priority: 30.03.88 GB 8807523

(43) Date of publication of application:
04.10.89 Bulletin 89/40

(84) Designated Contracting States:
AT BE CH DE ES FR GB GR IT LI LU NL SE

(71) Applicant: OXACO S.A.
2, Rue Charles-Bonnet
CH-1206 Genève (CH)

(72) Inventor: Jefford, Charles William Universite de Geneve
*Section de Chimie 30 Quai Ernest-Ansermet*
Ch-1211 Geneve 4 (CH)

(74) Representative: Ruffles, Graham Keith
MARKS & CLERK 57-60 Lincoln's Inn Fields
London WC2A 3LS (GB)

(54) **1,2,4-Trioxanes related to arteannuin.**

(57)
Aretannuin derivatives can be made by photo-oxygenation of a 2-oxabicyclo[4.4.0]dec-1,8a-ene compound using singlet oxygen, and reaction with a carbonyl compound $R^1R^2C=O$ to give a seco-desoxyarteannuin derivative substituted with $R^1$ and $R^2$ at the 2-position.

EP 0 335 717 A2

**Description**

## 1,2,4-Trioxanes Related to Arteannuin

BACKGROUND OF THE INVENTION

This invention relates to a new process for producing 1,2,4-trioxanes related to arteannuin, to the derivatives themselves, and to pharmaceutical uses of the derivatives.

Arteannuin, also known as Qinghaosu, is an antimalarial agent active against choloroquine-resistant strains of Plasmodium falciparum. It was isolated from the shrub Artemisia annua L (see for example Science (1985), 228, 1049), and has the structure:

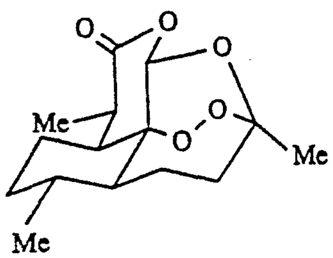

Synthesis is difficult and commercially impractical.

SUMMARY OF THE INVENTION

The present invention provides a new process opening up the possibility of synthesis of a family of useful arteannuin derivatives. For ease of reference, the arteannuin derivatives of this invention are named herein as derivatives of a parent compound "seco-desoxyarteannuin", which is here defined to have the structure:

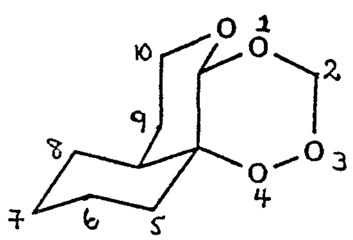

The process of this invention involves photo-oxygenation of a 2-oxabicyclo[4.4.0]dec-1,8a-ene compound using singlet oxygen, and reaction usually in situ with a carbonyl compound $R^1R^2C=O$ to give a seco-desoxyarteannuin derivative substituted with $R^1$ and $R^2$ at the 2-position. The process is of general applicability. For instance, the nature of the 2-substituent(s) is not critical. Moreover, the starting 2-oxabicyclo[4.4.0]dec-1,8a-ene compound may be substituted, giving seco-desoxyarteannuins correspondingly substituted at further positions, apart from the 2-position.

The present invention further provides compounds which may be made by the new synthetic route, that is, the seco-desoxyarteannuin derivatives substituted with $R^1$ and $R^2$ at the 2-position. The seco-desoxyarteannuin derivatives substituted with $R^1$ and $R^2$ at the 2-position represent a new class of fused derivatives of 1,2,4-trioxane, and typical compounds exhibit interesting pharmaceutical properties, which include, but are not restricted to, anti-malarial activity.

PREFERRED EMBODIMENTS OF THE INVENTION

The first step of the process involves photo-oxygenation using singlet oxygen of a 2-oxabicyclo[4.4.0]dec-1,8a-ene compound. The parent compound 2-oxabicyclo[4.4.0]dec-1,8a-ene itself is of the formula:

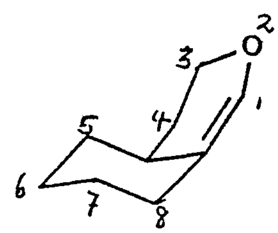

As mentioned above, the starting compound may be substituted, in order to produce seco-desoxyarteannuins correspondingly substituted at other than the 2-position. For example, the starting 2-oxabicyclo[4.4.0]dec-1,8a-ene compound can be 4,7-dimethyl-2-oxabicyclo[4.4.0]dec-1,8a-ene, of formula:

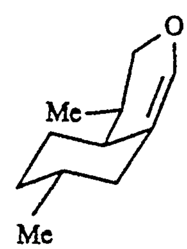

The photo-oxygenation is preferably effected at below 0°C, typicaly at -78°C, using singlet oxygen generated for example using irradiation of a solution of the reaction mix in methylene chloride further containing methylene blue as sensitizer. Methylene blue offers advantages for the present process in comparision with other sensitizers such as rose bengal.

In the process of this invention, the photo-oxygenation product is believed to be of the type

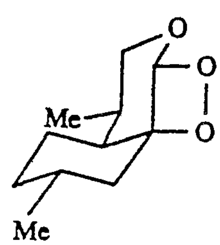

but is not ordinarily isolated. The photo-oxygenation

product is treated with an aldehyde, ketone or derivative thereof of the formula $R^1R^2C=O$, usually in the presence of a catalyst. This condensation is suitably effected with continued cooling in the presence of a catalyst such as trimethylsilyl trifluoromethanesulfonate (TMSOTf) or other silylsulfonates. Purely by way of illustration, $R^1$ and $R^2$ can be hydrogen, alkyl, aryl or functional groups; or $R^1$ with $R^2$ with the carbon to which they are attached can jointly be alicyclic.

Accordingly, in a preferred aspect of the invention, there are provided compounds of the general formula (I):

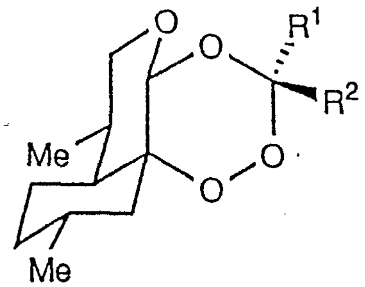

in which $R^1$ and $R^2$ can be hydrogen, alkyl, aryl or functional groups; or $R^1$ with $R^2$ with the carbon to which they are attached can jointly be alicyclic; though for anti-malarial activity, it seems that $R^1$ and $R^2$ can not both represent hydrogen. Such compounds are readily prepared by the process of this invention using (-)isopulegol as starting material, but compounds lacking the 6- and/or 9-methyl substituents, and compounds substituted in other positions can be made by suitable selection of another starting material.

The groups $R^1$ and $R^2$ can be the same or different alkyl groups, especially alkyl groups of 1 to 7, more preferably 1 to 4 carbon atoms, in particular methyl, ethyl or n-butyl, which are optionally substituted for example with one or more aryl groups such as phenyl; or with functional groups. Examples of functionally substituted alkyl groups include those of formula $C_nH_{2n}X$ where n is 1 to 7 and X represents an alkoxy group such as methoxy; hydroxy group; a halogen atom such as chlorine or fluorine; a carboxyl group which may be esterified or present as a salt such as a sodium or ammonium salt; an optionally alkyl-substituted amino group such as a diethylamino group; an alkoxycarbonyl group; a peroxide group; an ester (such as an optionally chiral oxycarbonyl, oxycarbonyloxy or oxyalkylcarbonyloxy ester); a carboxylic acid (such as an oxyalkylcarboxylic acid); a ketone; an imine; a hydrazone; an amino acid; a sulphonate residue; a peptide residue; a glycosyl group; a phosporyl group; a diphosphenyl group; or a phosphate residue.

The symbols $R^1$ and $R^2$ may represent the same or different aryl groups, which may be heterocyclic aryl groups, for instance with 5 or 6 ring atoms with 1 or 2 oxygen, sulphur or nitrogen heteroatoms. Examples of such aryl groups include a phenyl, p-tolyl, naphthyl or pyridyl group. The aryl groups can be substituted, for example, with one or more alkyl groups such as methyl; or with the substituents mentioned for alkyl groups.

The groups $R^1$ and $R^2$ can be the same or different functional groups, examples of which include the group X mentioned above.

$R^1$ and $R^2$ with the carbon atom to which they are attached can form an alicyclic group, optionally interrupted with O, S, S=O, $S(=O)_2$ or NH, and optionally substituted for example with the substituents illustrated for the alkyl groups, especially a cycloalkane of 3 to 7 carbons optionally interrupted with O or S, and optionally substituted with hydroxy or methyl, in particular a cyclopentane, cyclohexane or oxolane group.

The present invention embraces racemic mixtures of compounds of formula (I), as well as one or other of the enantiomeric forms. In general, the preferred compounds of this invention have the same conformation of the tricyclic ring as in arteannuin.

Further substitution is possible in the seco-desoxyarteannuin ring system. For instance, there may be substitution at the 5-position, typically with a substituent conforming with the definition for the group $R^1$. The preferred compounds lack the 10-oxo group of arteannuin, but synthesis of compounds with a 10-oxo group is not precluded.

The novel compounds of this invention mimic the architecture of arteannuin. Representative compounds are particularly active as antimalarial agents, more so than chloroquine or mefloquine, and commensurate with that of arteannuin itself. For example, testing by the method in Antimicrob Agents Chemother (1979), 16, 710, the $IC_{50}$ values for trioxanes 2,2,6,9-tetramethyl-seco-desoxyarteannuin and 6,9-dimethyl-seco-desoxyarteannuin-2-spirocyclopentane against the chloroquine-resistant Indochina W-2 clone of Plasmodium falciparum are 6.12 and 1.96 ng/ml. Arteannuin, chloroquine and quinine, as controls, gave an $IC_{50}$ of 0.78, 40.2 and 30.17 ng/ml, respectively. The present compounds therefore have activities close to that of arteannuin, and superior to that shown by traditional antimalarial remedies.

Furthermore, in view of other data, it is appears that the present compounds have general parasiticidal activity, including activity against protozoa and nematode parasites permitting use for instance in treatment of toxoplasmosis, pneumocystisis, leishmaniasis and onchocerciasis. In particular, the present compounds are of interest for the treatment of immmunocompromised individuals.

Hence, the present invention provides pharmaceutical compositions which comprise a compound of this invention, together with a pharmaceutically acceptable carrier.

The compounds of the present invention typically have low toxicity, permitting massive doses to be given to patients at the brink of death. For example, the compounds can be formulated as suspensions in oil and injected intramuscularly as a single shot. While formulations for parenteral administration may be used, it is within this invention to formulate the compounds as formulations for oral administration. In general, formulation of the compounds of this invention can be based on the conventional techniques available to the pharmacist. Tablets for prophylactic treatment are especially suitable. To

this end, some modifications of the compounds may be desirable, for example to enhance water solubility through inclusion of hydroxy, carboxyl, amino or other functional substituents. Suitable modifications can be achieved by routine experimentation. Indeed, an advantage of the present invention is that many different compounds can be obtained by a synthetic route.

The starting 2-oxabicyclo[4.4.0]dec-1,8a-ene compound can readily be prepared from a 2-oxabicyclo[4.4.0]decan-1-one, which in turn can be prepared from a 2-ethylenylcyclohexylcarboxylic acid. For example, (-)isopulegol of formula:

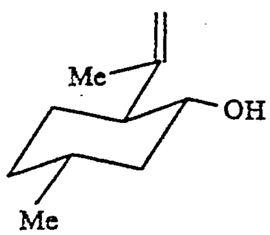

can be converted via its p-toluenesulfonate into the known 2-(isopropenyl)-5-methylcyclohexyl carboxylic acid

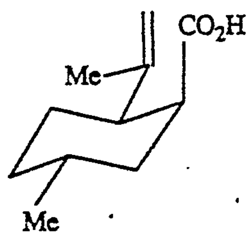

Oxidative hydroboration furnishes the lactone

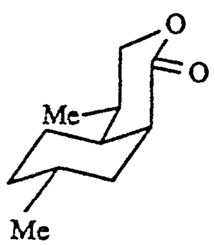

which can be reduced the lactol

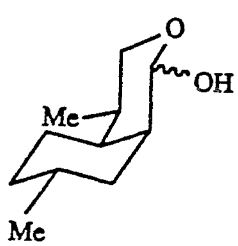

and dehydrated to the desired starting material, the 4,7-dimethyl-2-oxabicyclo[4.4.0]dec-1,8a-ene of formula given above,

None of these modifications alters the chirality of the starting material, and so the compound possesses three chiral centers which are the same as those in arteannuin.

Derivatization of the 2-substituted seco-desoxyarteannuin can be performed, which may for example involve chain formation to bridge substituents at the 2- and 5-positions, for example using carbene or radical insertion techniques. If desired, the ketone $R^1R^2C{=}O$ can be alicyclic and/or a pure optical isomer. The use of chiral ketones such as (-)camphor, carvone, pulegone or menthone enables stereospecific synthesis of a chiral product.

## EXAMPLES OF THE INVENTION

The present invention is illustrated by the following Examples for synthesis of new compounds.

### Example 1

(+)-4(R),7(R)-Dimethyl-2-oxa-4-$4_a$(S),$8_a$(R)-bicyclo[4.4.0]decan-l-one

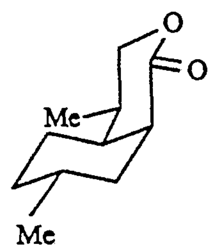

To a solution of 2-(isopropenyl)-5-methylcyclohexyl carboxylic acid (1.95 g, 10.7 mmol) (prepared in known manner from (-)-isopulegol in tetrahydrofuran (100 ml) was added dropwise 9-borabicyclo[3,3,1]nonane (9BBN) (Aldrich, 0.5M in hexane, 45 ml 22.5 mmol) under nitrogen. After stirring for 24 h, sodium hydroxide (6N, 10 ml) was added portionwise followed by slow addition of hydrogen peroxide (30%, 10 ml). The mixture was stirred at 50°C for 1.5 h and diluted with ethyl ether. The organic layer was washed twice with diluted brine. Next the aqueous layers were combined, acidified and extracted with ethyl ether. The ethereal solution was stirred overnight with a few crystals of p-toluenesulfonic acid ($TsOH.H_2O$). Evaporation followed by chromatography of the crude oil so obtained on silica gel gave the desired compound as a colorless oil, which formed crystals on standing (1.87g, 95%), mp 48-50°C.

$^1$H-NMR ($CDC1_3$) δ:
4.35 (d, d, J = 11, 4.2 Hz, 1H),
4.08 (d, d, J = 11, 3.8 Hz, 1H),
2.30 (d, m, J = 13 Hz, IH),
2.17 (d, t, J = 12, 3,5 Hz, 1H),
2.02 (m, 1H),
1.75 (d, m, 1H),
1.67 (m, 2H),
1.40 (m, 1H),
1.26 (d, q, J = 13, 3.8 Hz, 1H),
1.0-0.85 (m, 2H),
0.99 (d, J = 7.5 Hz, 3H),
0.91 (d, J = 6.5 Hz, 3H).

Example 2

4(R),7(R)-Dimethyl-2-oxa-$4_a$ (S)-bicyclo[4.4.0]dec-1,8a-ene

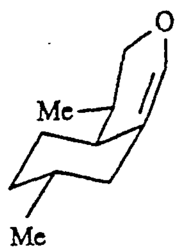

A solution of (+)4(R),7(R)dimethyl-2-oxa-$4_a$(S),$8_a$(R)bicyclo[4.4.0]decan-l-one (500 mg, 2.75 mmol) in toluene (100 ml) was reduced to 90 ml by distillation and then cooled to -70°C. Di-isobutylaluminum hydride (DIBAL) (1M in hexane, 6.8 ml, 2.5 equivalent) was added dropwise under nitrogen. The reaction was usually complete within 2 h as shown by TLC. The resulting mixture was poured into ice-sulfuric acid (2N) and extracted with ethyl ether. Drying ($Na_2SO_4$) and evaporation gave a residue which was dissolved in benzene (100 ml). The solution was heated under reflux with $TsOH.H_2O$ (15 mg) to remove water azeotropically. When the process was complete (as verified by TLC, 3 h), the solution was cooled and passed over a column of silica gel (10g). Evaporation gave pure product as a colorless oil, which solidified standing, (442 mg, 97%). A sample for analysis was obtained by short path evaporation (40°C/0.02 mm Hg), mp 35-37°C.

$^1$H-NMR ($CDCl_3$) δ:
6.2 (m, 1H)
3.72 (d, d, J = 10, 3 Hz, 1H)
3.52 (d, d J = 10, 8.5 Hz, 1H)
2.07 (m, 3H)
1.95 (m, 1H)
1.77 (m, 1H)
1.68 (m, 1H)
1.50 (t, m, J = 11 Hz, 1H)
1.34 (m, 1H)
1.13-0.85 (m, 3H)
0.87 (d, J = 4.5 Hz, 6H).

Example 3

2, 6, 9-trimethyl-seco-desoxyarteannuin

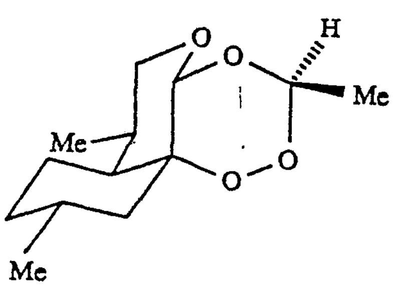

A solution of 4(R)-7(R)-dimethyl-2-oxa-$4_a$(S)-bicyclo[4.4.0]dec-1,8a-ene (80 mg, 0.48 mmol) and rose bengal (5 mg) in freshly distilled acetaldehyde (25 ml) was flushed with oxygen at -78°C under irradiation for 4 h. Irradiation was provided by a 500-W Sylvania FFX halogen-trungsten filament lamp screened with a UV cut-off filter (>418 nm). The progress of the reaction was monitored by TLC. The reaction solution was evaporated, the resulting residue diluted with dichloromethane (2 ml), pentane (2 ml) and filtered. The filtrate was evaporated to give crude product, which was then chromatographed on silica gel (eluent hexane:ethyl acetate, 20:1) to give, among other products, an inseparable 12:1 mix of the desired product and the 2-isomer.

major isomer, $^1$H-NMR ($CDCl_3$) δ:
5.93 (q, J = 5 Hz, 1H)
4.97 (s, 1H)
3.68 (d, d, d, J = 11.4 Hz, 1H)
3.55 (d, d, J = 11, 11 Hz, 1H)
2.79 (d, d, d, J = 13.4, 2 Hz, 1H)
2.35 (m, 1H)
1.75 (m, 1H)
1.55-1.33 (m, 3H)
1.21 (d, J = 5 Hz, 3H)
1.02 (d, d, J = 13, 13 Hz, 1H)
0.98-0.82 (m, 1H)
0.90 (d, J = 6.5 Hz, 3H)
0.67 (d, J = 7 Hz, 1H.

Example 4

2,2,6,9-tetramethyl-seco-desoxyarteannuin

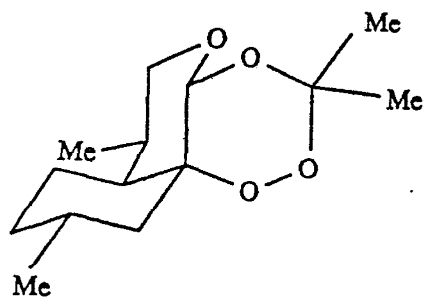

A solution of 4(R),7(R)-dimethyl-2-oxa-$4_a$(S)-bicyclo[4,4,0]dec-1,$8_a$-ene (52 mg, 0.31 mmol) and methylene blue (5 mg) in dry dichloromethane (25 ml) was cooled to -78°C and flushed with oxygen for 2.5 h under irradiation. Next acetone (1 ml) was added dropwise, followed by trimethylsilyl trifluoromethanesulfonate (0.065 ml). After stirring the mixture for 30 min., triethylamine (0.5 ml) was added. The resulting solution was poured into water and diluted with dichloromethane (20 ml). The organic layer was washed with water (3x, 10 ml) and dried ($Na_2SO_4$). After evaporation, the residue was purified on a silica gel column (eluent: hexane:ethyl acetate, 9.1) to give the desired trioxane (47 mg, 59%) as colorless crystals, mp 77-78°C (pentane).

$^1$H-NMR ($CDCl_3$) δ:
4.95 (s, 1H)
3.63 (d, d, d, J = 11, 4.5, 1 Hz, 1H)

3.43 (d, d, J = 11, 11 Hz, 1H)
2.67 (d, d, d, J = 13.5, 3.5, 2 Hz, 1H)
2.34 (m, 1H)
1.73 (s, 3H)
1.72-1.55 (m, 3H)
1.45-1.26 (m, 2H)
1.29 (s, 3H)
1.03 (d, d, J = 13, 13 Hz, 1H)
0.95-0.78 (m, 1H)
0.90 (d, J = 6.5 Hz, 3H)
0.68 (d, J = 7 Hz, 3H).

MS m/e: 238 ($M^+$), 191, 167, (100%), 149, 123, 109, 95, 81, 69, 55.

The structure was determined by X-ray. It had the same absolute configuration as the corresponding part of arteannuin.

Example 5

6,9-dimethyl-seco-desoxyarteannuin-2-spirocyclopentane

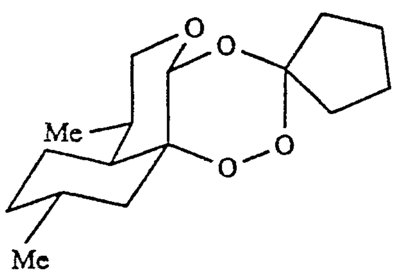

A solution of 4(R),7(R)-dimethyl-2-oxa-$4_a$(S)-bicyclo[4.4.0]dec-1,$8_a$-ene (16, 100 mg, 0.6 mmol) in dichloromethane (50 ml) containing methylene blue (7 mg) was cooled and flushed with oxygen under irradiation for 3 h. Cyclopentanone (1.3 ml, excess) was added slowly, followed by trimethylsilyl trifluoromethanesulfonate (0.2 ml). The resulting solution was stirred at this temperature for 30 min. Triethylamine (1 ml) was added and the solution was poured into water and the mixture was diluted with dichloromethane. The organic layer was washed with water (3x, 20 ml) and dried ($NaSO_4$). After evaporation, the crude product was purified by chromatography on silica gel column (eluent, hexane:ethyl acetate, 30:1) to give the desired trioxane (104 mg, 61%) as colorless crystals. Recrystallization from cold pentane afforded a sample for analysis, mp 83-85°C.

$^1$H-NMR, ($CDCl_3$) δ:
4.94 (s, 1H)
3.63 (d, d, d, J = 13, 5, 1 Hz, 1H)
3.44 (d, d, J = 13, 13 Hz, 1H)
2.75 (d, d, d, J = 14, 4, 2 Hz, 1H)
2.64 (m, 1H)
2.32 (m, 1H)
1.94-1.50 (m, 10H)
1.45-1.25 (m, 2H)
1.02 (d, d, J = 13, 13 Hz, 1H)
0.94-0.80 (m, 1H)
0.90 (d, J = 6.5 Hz, 3H)
0.67 (d, J = 7 Hz, 3H)

MS m/e: 166 (- $O_2$, - cyclopentanone), 137, 112 (100%), 97, 84, 69, 55.

## Claims

1. A process for preparing a 2-substituted seco-desoxyarteannuin, which process comprises photo-oxygenation of a 2-oxabicyclo[4.4.0]dec-1,8a-ene compound using singlet oxygen, and reaction with a carbonyl compound $R^1R^2C=O$ to give a seco-desoxyarteannuin derivative substituted with $R^1$ and $R^2$ at the 2-position.

2. A process according to claim 1, in which the photo-oxygenation is performed in methylene chloride using methylene blue as sensitizer.

3. A seco-desoxyarteannuin derivative substituted with one or two substituents at the 2-position.

4. A compound of the general formula (I):

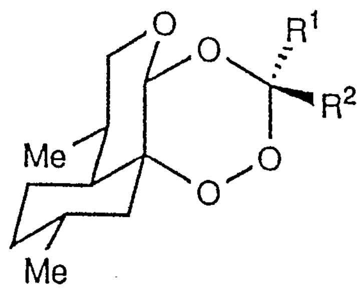

in which $R^1$ and $R^2$ are hydrogen, alkyl, aryl or functional groups; or $R^1$ with $R^2$ with the carbon to which they are attached jointly form an alicyclic ring; provided that $R^1$ and $R^2$ are not both hydrogen.

5. A pharmaceutical composition which comprises a 2-substituted seco-desoxyarteannuin derivative, together with a pharmaceutically acceptable carrier.